# EUROPEAN PATENT APPLICATION

(11) **EP 3 400 916 A1**
(43) Date of publication of application: **14.11.2018**
(21) Application number: 16882906.7
(22) Date of filing: 06.01.2016
(51) Int. Cl.: A61F 13/02

(54) **ADHESIVE COVERING STRUCTURE**

(71) Applicant: Easting Biotech Company Limited, New Taipei City 241 (TW)
(72) Inventor: LIN, Tsu-Tai, New Taipei City 241 (TW)
(74) Representative: Lang, Christian
(86) International application number: PCT/CN2016/070321
(87) International publication number: WO 2017/117761

(57) **Abstract**

The present invention is related to a breathable, stretchable, antibacterial and highly absorptive adhesive covering structure, which comprises a melt-blown nonwoven fabric layer with multi-directional elasticity disposed on the top; an interpenetrating network polymer layer disposed beneath the melt-blown nonwoven fabric layer; and a hydrogel layer disposed beneath the interpenetrating network polymer layer, wherein the interpenetrating network polymer layer is formed by the copolymerization of a part of the melt-blown nonwoven fabric layer and a part of the hydrogel layer, and the basis weight of the melt-blown nonwoven fabric layer in the adhesive covering structure is between 20 g/m² to 40 g/m².

## Description

### FIELD OF INVENTION

The present invention is related to an adhesive covering structure, particularly a breathable, stretchable, elastic, antibacterial and absorptive adhesive covering structure.

### BACKGROUND OF INVENTION

According to recent scientific and statistical reports, the demand of the global wound market is quickly increasing, especially surgical wounds which have reached up to more than 100,000,000 wounds per year and is still increasing. Tearing wounds caused by accidents hover around 20,000,000 per year with burn wounds and chronic disease, diabetes and age related ulcerative wounds exceeding 10,000,000 and 30,000,000 respectively.

Due to the increase demands of surgical treatments and endoscopic surgery, the risk of surgery has greatly decreased, but subsequently, the demands of better wound care and scar prevention is on the rise. There is currently a diversity of wound adhesive covering structures on the market to accelerate reducing time of wound healing and scar elimination.

Conventional adhesive covering structures are formed from natural plant fibers or animal furs such as gauzes, cotton pads, wool and etc. These types of adhesive covering structures only provide temporary coverage and should be replaced after a certain period of time. However, these adhesive covering structures easily stick to the wound openings and tear down newly healed skin. Thus, the removal of the adhesive covering structures agonizes the patients and is disadvantageous to the recover process of wounds.

Recent studies on wound adhesive covering structures give further scientific insight into wound care. Results show that an optimal wound adhesive covering structure should provide a suitable environment for cell growth and regeneration in the wound where exudate is absorbed and controlled; be breathable, permeable to moisture and preventable to bacteria; adhere smoothly to the body; allow carriage and administration of drugs; have good compatibility with tissue such that removal will not result in tearing of new-grown skin; have tensile strength; and be user friendly.

In comparison to conventional adhesive covering structures, hydrogel types of adhesive covering structures are better in aspects of breathability and moisture permeability and the ability to prevent bacterial invasion and control exudate, rendering it a suitable environment for cell growth and regeneration in the wound. Additionally, hydrogel types of adhesive covering structures are able to adhere smoothly to wound openings and tend not to cause secondary damage through tearing of new-grown skin upon removal. Therefore, hydrogel types of adhesive covering structures are used widely in the biomedical field.

The supporting layer of conventional adhesive covering structure products are usually made from less elastic materials such as polyethylene (PE), polypropylene (PP) or nylon. Therefore, when combined with hydrogels to form wound adhesive covering structure products, the structure of it is prone to breakage due to the inelastic supporting layer. This results in not being able to adhere smoothly to the body, therefore likely to lead to discomfort. Moreover, lacking conformity to user body causes the hydrogel type adhesive covering structure low efficiency of liquid absorption, distribution and maintenance. Furthermore, another problem likely to arise from the low stretching ability the possible result of the breakage of hydrogel structures into independent and isolated groups of material, which not only causes discomfort but also hampers the covering efficiency of the hydrogel.

There is an ongoing need to develop a hydrogel adhesive covering structure that exceeds the performance of the current structure in the field. More particularly, an adhesive covering structure that is stretchable; is able to adhere smoothly to the body; and has the ability to efficiently and constantly absorb exudates.

### DESCRIPTION OF INVENTION

After multiple experiments, the inventors discovered that adjusting the materials and the basis weight of the supporting layer could improve the aforementioned problems. If the basis weight of the supporting layer is too high, the hydrogel layer could fall apart due to the hydrophobicity of the supporting layer influencing the ability for the hydrogel layer to integrate with the supporting layer. Moreover, a high basis weight renders the adhesive covering structure non-transparent, making it difficult to use. If the basis weight of the supporting layer is too low, the tensile strength and the stretch ratio would be lower than expectation and the hydrogel layer would be more likely to fall apart from the waterproof covering structure after losing stickiness from water absorption. The present invention is related to a breathable, stretchable, antibacterial and highly absorptive adhesive covering structure, which comprises a melt-blown nonwoven fabric layer with multi-directional elasticity disposed on the top; an interpenetrating network polymer layer disposed beneath the melt-blown nonwoven fabric layer; and a hydrogel layer disposed beneath the interpenetrating network polymer layer, wherein the interpenetrating network polymer layer is formed by the copolymerization of a part of the melt-blown nonwoven fabric layer and a part of the hydrogel layer and the basis weight of the melt-blown nonwoven fabric layer in the adhesive covering structure is between 20 g/m² to 40 g/m².

An embodiment of the present invention, the melt-blown nonwoven fabric layer has a basis weight of 20 g/m² to 40 g/m². In another preferred embodiment of the present invention, the melt-blown nonwoven fabric layer has a basis weight of 20 g/m² to 35 g/m². In another preferred embodiment of the present invention, the melt-blown nonwoven fabric layer has a basis weight of 20 g/m² to 30 g/m², such that the adhesive covering structure is transparent.

According to the adhesive covering structure, in a preferred embodiment, the copolymer includes, but is not limited to chemical polymerization, wherein the chemical polymerization includes, but is not limited to formation from UV light exposure. Additionally, the melt-blown nonwoven fabric part of the interpenetrating network polymer layer is hydrophobic and its fiber is partially exposed. In another preferred embodiment, the hydrogel layer is capable of excess water and wound exudate absorption, such that the skin area covered by it is moisturized. The hydrogel layer comprises (a) monomer; (b) plasticizer; (c) photoinitiaters; (d) cross-linkers; and (e) thickeners.

In a preferred embodiment, the weight ratios of the hydrogel layer are as follows:

| | |
|---|---|
| Acrylic monomer | 15-30 units |
| Acrylic sulfonate monomer | 10-50 units |
| Glycerin | 15-45 units |
| Photoinitiator | 0.01-0.1 units |
| Unsaturated double bonded ester bifunctional monomers | 0.01-0.2 units |

In a preferred embodiment, the monomer includes acrylic monomer and acrylic sulfonate monomer, wherein the acrylic sulfonate monomer provides the hydrogel layer antibacterial properties; glycerin functions as the plasticizer and the thickener, where glycerin provides the hydrogel layer soft and hydrophilic properties; the photoinitiator produces free radicals and completes polymerization under 1-50 seconds of UV light exposure where it becomes non-toxic to cells postreaction; and the unsaturated double bonded ester bifunctional monomers functions as the crosslinker.

According to the present invention, the adhesive covering structure further comprises a bioactive non-woven fabric layer, where it is disposed under the hydrogel layer. In a preferred embodiment, the bioactive non-woven fabric layer is made of collagen and chitin. In another embodiment, the adhesive covering structure further comprises a polyurethane film where it is a one-way permeable membrane with a hydrophobic pressure-sensing gel layer thereon. The structural order of the adhesive covering structure from top to bottom is the polyurethane film, the hydrophobic pressure-sensing gel layer, the melt-blown nonwoven fabric layer, the interpenetrating network polymer layer and the hydrogel layer.

According to the present invention, in a preferred embodiment, the water absorption rate is 10-40g/g. The term "water absorption rate" is defined as the amount of water a gram of the present invention can absorb in grams. The melt-blown nonwoven fabric layer is made of thermoplastic polyurethane (hereinafter referred as TPU).

In a preferred embodiment of the present invention, the hydrogel layer contacts the wound area, where the hydrogel layer maintains the optimal moisture for the acceleration of wound healing. A moisturized environment is beneficial to wound healing because cells cannot survive under dehydrated conditions. Although other moisturized gauzes or bandages can also provide a moisture environment but these adhesive covering structures require frequent dressing changes, thus resulting in possible damage of new grown cells and bear the risk of dehydration. The hydrogel layer in the present invention will reduce stickiness upon liquid absorption, therefore it will cause less wound damage compared to moisturized gauzes or bandages during wound dressing changes. In addition, its water absorption rate is more compatible to the degree of optimal moisture for wound healing. It absorbs excess liquid, maintains optimal skin moisture and has drug deliver capability.

The present invention is applicable to multiple wound adhesive covering structures, dressing for post-circumcision wounds, electrode adhesive covering structures, face masks or cosmetic adhesive covering structures, or other daily sticker products such as mosquito stickers and heel stickers. The beneficial effect of the present invention is that it possesses multi-directional elasticity and is suitable for a variety of wound adhesive covering structures where it provides a better wound healing environment, decreases duration of wound-healing and lowers infection possibilities.

### BREIF DESCRIPTION OF THE DRAWINGS

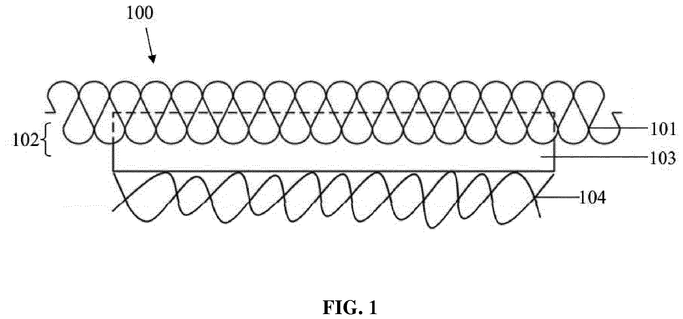

**FIG. 1** is the schematic diagram of the adhesive covering structure of the present invention.

In the figure, the adhesive covering structure is referred to as **100,** the melt-blown nonwoven fabric layer is referred to as **101,** the interpenetrating network polymer layer is referred to as **102,** the hydrogel layer is referred to as **103,** and the bioactive nonwoven fabric layer is referred to as **104.**

### DETALED DESCRITION OF THE EMBODIMENTS

A preferred embodiment of the present invention is described in detail as follows where the figure number is identical to the drawings and is served as a reference. It is noted that the following embodiments is not to limit the invention to a particular form, rather to serve as a representative of the multiple configurations of the present invention.

**FIG. 1** is the schematic diagram of the adhesive covering structure of the present invention. The present invention **100** includes: a melt-blown nonwoven fabric layer **101** with multi-directional elasticity on the top and a basis weight between 20 g/m² to 40 g/m²; an interpenetrating network polymer layer **102** disposed beneath the melt-blown nonwoven fabric layer **101**; and a hydrogel layer **103** disposed beneath the interpenetrating network polymer layer **102;** where the interpenetrating network polymer layer **102** is formed by the copolymerization of a part of the melt-blown nonwoven fabric layer **101** and a part of the hydrogel layer **103.**

According to the present invention **100,** the adhesive covering structure **100** further comprises a bioactive nonwoven fabric layer **104** disposed beneath the hydrogel layer **103.** In one embodiment, the melt-blown nonwoven fabric layer **101** is made of thermoplastic polyurethane and the bioactive nonwoven fabric layer **104** is made of collagen or chitin.

### Preparation of Hydrogen gel layer:

(a) Provide a mixture comprising the following steps:
   (I) Mix the photoinitiator with the acrylic monomer until dissolve;
   (II) Add glycerin and mix until dissolve;
   (III) Add acrylic sulfonate monomer and mix until dissolve;
   (IV) Add and mix glycerin.
(b) Addition of a mixture comprising the following steps:
   (I) Mix photoinitiator with the unsaturated double bonded ester bifunctional monomers.
(c) Mix mixtures from step (a) and step (b).
(d) Cover the mixture made from step (c) on a melt-blown nonwoven fabric (e.g.TPU), and when mixture made from step (c) permeates the melt-blown nonwoven fabric, expose it under UV light so as to carry out crosslink polymerization reaction to obtain adhesive covering structure **100.**

The composite ratios of the hydrogel layer are as follows:

| | |
|---|---|
| Acrylic monomer | 15-30 units |
| Acrylic sulfonate monomer | 10-50 units |
| Glycerin | 15-45 units |
| Photoinitiator | 0.01-0.1 units |
| Unsaturated double bonded ester bifunctional monomers | 0.01-0.2 units |

The information of the present invention is as follows:

| Name | TPU Thermoplastic Elastomer Nonwoven | | |
|---|---|---|---|
| Specifications | 30g(30gsm/m²)*360mm (Width) | | |
| Test Items | | Test Specifications | Units |
| Width | | 355.0 ∼ 365.0 | mm |
| Average Basis Weight | | 27.0 ∼ 33.0 | g/m² |
| Average Thickness | | 0.11 ∼ 0.14 | mm |
| Maximum Longitudinal Tensile Strength | | 0.23 ∼ 0.31 | Kgf |
| Maximum Transverse Tensile Strength | | 0.24 ∼ 0.28 | Kgf |
| Maximum Longitudinal Elasticity | | 100∼200 | % |
| Maximum Transverse Elasticity | | 160∼240 | % |

The present invention has a water absorption rate of 10-40g/g. The term "water absorption rate" is defined as the amount of water a gram of the present invention can absorb in grams. The measurement of "water absorption rate" is as follows. The measurement of water absorption rate is as follows: dry the present invention, cut it into suitable sample sizes and measure mass. Then, put the samples in deionized water, wait for absorption to reach equilibrium, remove from deionized water and let it rest. After removing excess water from the surface using filter paper, measure mass. The calculation of "water absorption rate" is: water absorption rate = (w2 - wl)/wl , where w1 is the dry mass of the adhesive covering structure (unit: g) and w2 is the mass of the adhesive covering structure when water absorption reaches equilibrium.

Although the present invention is described as the above embodiment, the description is not to limit the invention. Any skilled person in the art is capable of adjusting the present invention without straying from the spirit or scope thereof, thus the extent of the patent protection is determined by the patent claims.

## Claims

1. An adhesive covering structure, comprising:
a melt-blown nonwoven fabric layer with multi-directional elasticity disposed on the top;
an interpenetrating network polymer layer disposed beneath the melt-blown nonwoven fabric layer; and
a hydrogel layer disposed beneath the interpenetrating network polymer layer,
wherein the interpenetrating network polymer layer is formed by the copolymerization of a part of the melt-blown nonwoven fabric layer and a part of the hydrogel layer, and
the basis weight of the melt-blown nonwoven fabric layer in the adhesive covering structure is between 20 g/m² to 40 g/m².

2. The adhesive covering structure of claim 1, which further comprises a bioactive nonwoven fabric layer adhesively disposed beneath the hydrogel layer.

3. The adhesive covering structure of claim 1, which has a water absorption rate of 10-40g/g.

4. The adhesive covering structure of claim 1, wherein the melt-blown nonwoven fabric layer is made of thermoplastic polyurethane.

5. The adhesive covering structure of claim 2, wherein the bioactive nonwoven fabric layer is made of collagen or chitin.

6. The adhesive covering structure of claim 1, wherein the copolymerization is a chemical polymerization.
